# EUROPEAN PATENT SPECIFICATION

(11) **EP 2 079 512 B1**
(45) Date of publication and mention of the grant of the patent: **23.06.2010**
(21) Application number: 06812931.1
(22) Date of filing: 24.10.2006
(51) Int. Cl.: A61N 1/05

(54) **CONNECTING DEVICE AND METHOD FOR MANUFACTURING THE SAME**
VERBINDUNGSVORRICHTUNG UND VERFAHREN ZU IHRER HERSTELLUNG
DISPOSITIF DE CONNEXION ET PROCÉDÉ DE FABRICATION DE CELUI-CI

(43) Date of publication of application: 22.07.2009
(73) Proprietor: St. Jude Medical AB, 175 84 Järfälla (SE)
(72) Inventor: JARL, Per, 175 69 Järfälla (SE); HILL, Rolf, 175 55 Järfälla (SE)
(86) International application number: PCT/SE2006/001204
(87) International publication number: WO 2008/051118

(56) References cited:
- FR-A- 2 724 566
- US-A- 5 837 006
- US-A1- 2002 188 340
- US-A1- 2002 193 860
- US-A1- 2005 251 240

## Description

The description relates to a connecting device for connection of a medical implantable lead to tissue inside a body, comprising a helix being adapted to be screwed out from a header sleeve at a distal end of the lead and into the tissue, and a shaft which carries the helix and is rotatably journalled in the lead and at the same time displaceable to extend the helix into the tissue.

The invention relates to a method for manufacturing of such a connecting device.

### Background of the invention

For controlling and/or monitoring of an arbitrary organ inside a human or animal body, e.g. a heart by means of a pacemaker, it is common practice to attach an electrical lead to the organ for transmitting and receiving electrical signals to or from the organ. A common way to accomplish the attachment of the lead to the tissue, is to screw out a helix from a so called header sleeve at a distal end of the lead and into the tissue. The helix will then normally function both as the attachment and as an electrode to transmit and receive the electrical signals.

To accomplish this, the helix is attached to a shaft inside the lead which is rotatably journalled in the lead and displaceable in the longitudinal direction. Such an arrangement will allow rotation and extending of the helix out from the tip of the lead.

One example of a prior art connecting device is disclosed in US 2005/0251240 A1. Here, the helix is attached to a shaft by being screwed onto windings formed in the shaft. By rotating the shaft by means of for example a stylet inserted through the lead, the helix as well as the shaft will be screwed in the outward direction from the lead by engagement of the helix windings with a knob on the inside of a header sleeve where the helix is accommodated.

Another example of a prior art connecting device is disclosed in US 5837006, where a coiled wire conductor together with a movable stop member functions as a shaft. The helix is attached on the distal end of the stop member and thread around a guide mechanism, such when rotating the wire conductor, the helix together with the stop member and the wire conductor, will be screwed out and advanced forward through the sleeve.

However, there are several disadvantages associated with prior art connecting devices of the above designated kind. The connecting devices used today are composed of at least two components, i.e. a shaft and a helix, that needs to be aligned with each other within tight tolerances and both are complex and expensive to manufacture and a separate assembling process is necessary to accomplish the assembling. The components need also be electrical as well as mechanical connected to each other in a secure and reliable way. In the assembling process of the shaft and the helix it is always a risk that the two components will become misaligned, which is a common reason for rejection with increased costs as a consequence. It is also a risk for poor mechanical and/or electrical contact between the helix and the shaft when assembling them together.

FR 2724566 discloses a cardiac probe having a distal electrode and comprising a helix for anchoring the probe at a cardiac tissue. The helix and a helix shaft are made in one unitary piece.

### Summary of the invention

It is an object of the invention to reduce the disadvantages with prior art connecting devices. More precisely it is an object of the invention to facilitate and reduce costs for manufacture of the connecting device as well as facilitate assembling of the medical implantable lead.

The invention relates to a method for manufacturing of a connecting device according to claim 1.

The invention is thus based on the understanding that the above object may be achieved by making the connecting device in one piece, i.e. the helix and the shaft being integrated and manufactured in the same manufacturing process. In this way it is possible to completely eliminate any misalignment problems between the shaft and the helix. The complexity of the manufacture will also be reduced since any attachment area between the shaft and the helix can be dispensed with as well as the assembly process. Moreover, there is no risk of insufficient mechanical or electrical contact between the helix and the shaft.

The combined shaft and helix is manufactured originating from a blank, which may be a little longer in comparison with prior art shafts, which is formed in accordance with the outer shape of the finished connecting device and then to form a hole or bore a distance along the centre line from the distal end of the connecting device. Subsequently, the helix windings are formed by any suitable material removing process from the outer surface into the bore. The material removing process could preferably be performed by electro erosion or laser cutting, but it can also be done by e.g. milling, water jet cutting or etching. However, it is also possible to manufacture the connecting device by for example moulding or sintering, possible in combination with suitably finishing for obtaining surfaces with high quality.

One advantage with forming the helix windings by a material removing process, is that the form of the helix easily can be altered according to special desires or requirements. For example, the helix can be made with a variable cross section over its length, with projections or the like. On the whole, the invention makes it easier to perform modifications of the shape of the helix windings since it will be only one component that has to be altered instead of at least two in case of a prior art connecting device, assembled from a separate shaft and helix, in which also the mating part of the shaft has to be altered.

Sometimes it is desirable to improve and/or adjust the impedance of the helix surface by coating at least some portions of the surface with an electrical insulating material. By means of the material removing process according to the invention, this selective coating can be facilitated. E.g. when forming the helix of an electrically conducting material, the outer surface of the blank as well as, if desirable, also the inner surface of the bore, can easily be coated with an electrically insulating material, such that when the helix windings are formed by a material removing process, the cut edge surfaces of the helix windings will be electrically conductive. On the other hand, when forming the helix of an electrically insulating material, the outer surface of the blank as well as, if desirable, also the inner surface of the bore, can easily be coated with an electrically conducting material, such that when the helix windings are formed by a material removing process, the cut edge surfaces of the helix winding will be electrically insulated.

### Brief description of the drawings

The invention will hereinafter be described by reference to the accompanying drawings, in which:
- Fig 1: is a perspective view of a prior art connecting device comprising an assembled shaft and helix;
- Fig 2: is a perspective view of an integrated connecting device according to the invention;
- Fig 3: is a cut elevation showing the connecting device according to fig 3 inserted into a header sleeve of a medical implantable lead and being in a retracted state;
- Fig 4: is a cut elevation according to fig 3 showing the connecting device in a projected state; and
- Fig 5-9: are perspective views illustrating manufacturing steps in sequence according to an embodiment of the invention for manufacturing the connecting device.

### Detailed description of prior art and one embodiment of the invention

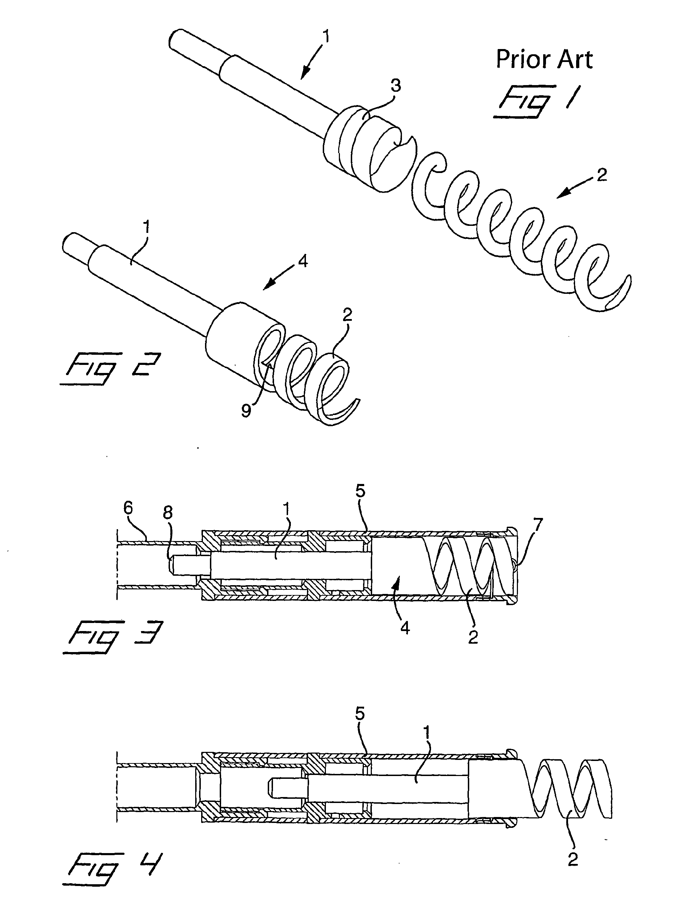

Reference is first made to fig 1, in which an embodiment of a prior art connecting device is shown. From the perspective view in fig 1 is seen that the connecting device is assembled of a shaft 1 and a helix 2 in form of separate components, wherein the helix is attached to the shaft by way of the most proximal turns of the helix mating and being in engagement with helical grooves 3 being formed in the distal end of the shaft. This has to effect that the shaft and the helix has to be manufactured in separate manufacturing processes and then being assembled into one unit under consideration of proper alignment of and electrical contact between the shaft and the helix in relation to each other. This necessitates a separate attachment process, such as welding, and suitable adaptation measures, such as forming of the grooves 3, to make the shaft and helix fit together.

Now reference is made to fig 2 of the drawings, in which an embodiment of a connecting device is shown in a perspective view. The shaft and the helix are integrated and manufactured into a connecting device 4 in one unitary piece, having a shaft portion 1 and a helix portion 2. Accordingly, there is no need for forming of helical grooves in the distal portion of the shaft for receiving the most proximal turns of the helix windings.

In the cut elevations of fig 3 and 4, the connecting device is shown positioned in a header sleeve 5 at a distal end of a medical implantable lead 6, in a retracted as well as a projected state, respectively. The medical implantable lead has a sufficient length for its intended use and typically the length is between 50-100 cm. A knob 7 on the inside of the header sleeve, is in engagement with the helical windings of the helix 2 such that when rotating the integrated connecting device 4, which e.g. can be performed by means of a not shown stylet inserted into the lead 6 and being in engagement with an engaging formation in the proximal end of the shaft portion 1, the helix will be screwed out from an opening in the distal end of the header sleeve. The rotation and screwing out of the helix in relation to the header sleeve 5, is allowed by the shaft being rotatably and displaceably journalled in the header sleeve 5. The knob 7 also functions as a stop member for restricting the maximum projection of the helix from the distal end of the header sleeve 5 in that the knob will abut against a shoulder 9 formed innermost of the helix windings.

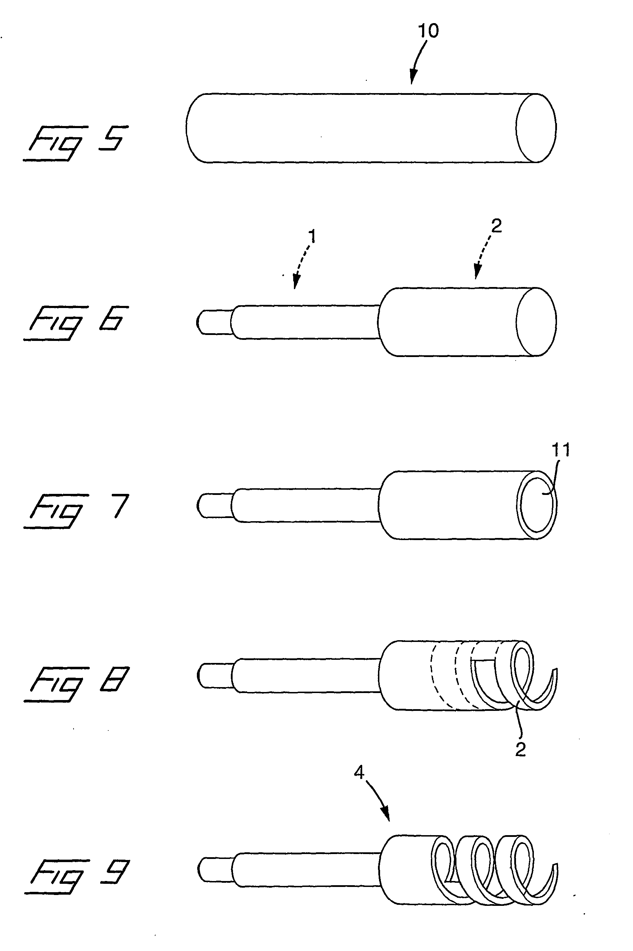

In figs 5 to 9 is illustrated a manufacturing method in sequential steps for manufacturing of an integrated connecting device according to one embodiment of the invention.

In fig 5 is shown an initial blank 10, in form of a cylinder, to be formed into a connecting device. The material in the blank can be any suitable, e.g. a metal such as platinum iridium or tantalum, ceramics or plastics.

Subsequently, the outer contour of the connecting device is formed in the blank according to fig 6.

In a next step, according to fig 7, a hole or bore 11 is formed, e.g. by drilling, concentric in the longitudinal direction from the distal end of the blank and a desired distance into it. This bore will form the inner bore of the finished helix.

In a last step, according to fig 8, the windings of the helix portion 2 are formed by a material removing shaping, e.g. by electro erosion, by removing of material from the outer surface, through the blank and into the bore. When this is done an integrated, unitary connecting device is formed according to fig 9.

## Claims

1. Method for manufacturing a connecting device (4) for connection of a medical implantable lead (6) to tissue inside a body, comprising a helix (2) being adapted to be screwed out from a header sleeve (5) at a distal end of the lead and into the tissue, and a shaft (1) which carries the helix (2) and is rotatably journalled in the lead (6) and at the same time displaceable to extend the helix (2) into the tissue, comprising manufacturing the shaft (1) and the helix (2) integrated in one unitary piece, **characterized in that** manufacturing the shaft (1) and the helix (2) integrated in one unitary piece comprises:
providing an elongated blank (10);
forming the blank (10) according to the desired outside shape of the shaft (1) and the helix (2);
forming an elongated bore (11) concentric in a longitudinally direction a desired distance into the blank (10) from its distal end; and
forming helically threads of the helix (2) by a material removing processing of the blank (10) from the outer surface, through the material and into the inner bore.

2. Method according to claim 1, **characterized by** forming the helically threads of the helix (2) by means of electro erosion.

3. Method according to claim 1 or 2, **characterized by** forming the helix of an electrically conducting material and coating the outer surface of the blank (10) with an electrically insulating material.

4. Method according to claim 3, **characterized by** coating the inner surface of the bore (11) with the electrically insulating material, such that when the helically threads of the helix (2) are formed by the material removing processing cut edge surfaces of the helically threads will be electrically conductive.

## Patentansprüche

1. Verfahren zum Herstellen einer Verbindungsvorrichtung (4) zum Verbinden einer medizinischen implantierbaren Leitung (6) mit Gewebe im Innern eines Körpers, umfassend eine Spirale (2), die angepasst ist, von einer Kopfhülse (5) an einem distalen Ende der Leitung heraus und in das Gewebe geschraubt zu werden, und einen Schaft (1) der die Spirale (2) trägt und in der Leitung (6) drehbar zapfengelagert und gleichzeitig verschiebbar ist, um die Spirale (2) in das Gewebe zu verlängern, umfassend das Herstellen des Schafts (1) und der Spirale (2), die in einem einzigen Stück integriert sind, **dadurch gekennzeichnet, dass** das Herstellen des Schafts (1) und der Spirale (2), die in einem einzigen Stück integriert sind, umfasst:
Bereitstellen eines Rohlings (10);
Bilden des Rohlings (10) gemäß der gewünschten Außenform des Schafts (1) und der Spirale (2);
Bilden einer länglichen Bohrung (11), die von seinem distalen Ende aus einen gewünschten Abstand in den Rohling (10) in einer Längsrichtung konzentrisch ist; und
Bilden spiralförmiger Gewinde der Spirale (2) durch eine Materialabtragungsbearbeitung des Rohlings (10) von der Außenfläche durch das Material und in die innere Bohrung.

2. Verfahren nach Anspruch 1, **gekennzeichnet durch** Bilden der spiralförmigen Gewinde der Spirale (2) mittels Elektroerosion.

3. Verfahren nach Anspruch 1 oder 2, **gekennzeichnet durch** Bilden der Spirale eines elektrisch leitenden Materials und Beschichten der Außenfläche des Rohlings (10) mit einem elektrisch isolierenden Material.

4. Verfahren nach Anspruch 3, **gekennzeichnet durch** Beschichten der Innenfläche der Bohrung (11) mit dem elektrisch isolierenden Material, so dass, wenn die spiralförmigen Gewinde der Spirale (2) **durch** die Materialabtragungsverarbeitung gebildet werden, Schneidkanten der spiralförmigen Gewinde elektrisch leitend werden.

## Revendications

1. Procédé de fabrication d'un dispositif de connexion (4) pour la connexion d'un conducteur médical implantable (6) à un tissu à l'intérieur d'un corps, comprenant une hélice (2) adaptée pour être dévissée hors d'un manchon de collecteur (5) à une extrémité distale du conducteur et dans le tissu, et une tige (1) qui porte l'hélice (2) et est supportée de manière rotative dans le conducteur (6) et en même temps est déplaçable pour étendre l'hélice (2) dans le tissu, comprenant la fabrication de la tige (1) et de l'hélice (2) intégrées en une pièce unique, **caractérisé en ce que** la fabrication de la tige (1) et de l'hélice (2) intégrées en une pièce unique comprend :
fournir une préforme allongée (10) ;
former la préforme (10) selon la forme extérieure souhaitée de la tige (1) et de l'hélice (2) ;
former un trou allongé (11) concentrique dans une direction longitudinale sur une distance souhaitée dans la préforme (10) depuis son extrémité distale ; et
former des filets hélicoïdaux de l'hélice (2) par une opération d'enlèvement de matériau de la préforme (10) depuis la surface extérieure, à travers le matériau et dans le trou intérieur.

2. Procédé selon la revendication 1, **caractérisé par** le formage des filets hélicoïdaux de l'hélice (2) au moyen d'électro-érosion.

3. Procédé selon la revendication 1 ou 2, **caractérisé par** le formage de l'hélice d'un matériau électriquement conducteur et revêtement de la surface extérieure de la préforme (10) avec un matériau électriquement isolant.

4. Procédé selon la revendication 3, **caractérisé par** le revêtement de la surface intérieure du trou (11) avec le matériau électriquement isolant, de sorte que lorsque les filets hélicoïdaux de l'hélice (2) sont formés par l'opération d'enlèvement de matériau, des surfaces d'arête de coupe des filets hélicoïdaux seront électriquement conductrices.
